# EUROPEAN PATENT SPECIFICATION

(11) **EP 2 906 529 B1**
(45) Date of publication and mention of the grant of the patent: **23.11.2016**
(21) Application number: 13782934.7
(22) Date of filing: 14.10.2013
(51) Int. Cl.: C07C 227/10, C07C 227/16, C07C 229/34, C07D 215/56

(54) **A NEW PRODUCTION METHOD AND NEW INTERMEDIATES OF SYNTHESIS OF ELVITEGRAVIR**
VERFAHREN ZUR HERSTELLUNG VON ELVITEGRAVIR UND ZWISCHENPRODUKTE DAFÜR
PROCÉDÉ ET INTERMÉDIAIRES POUR LA SYNTHÈSE DE L'ELVITEGRAVIR

(30) Priority: 12.10.2012 CZ 20120697
(43) Date of publication of application: 19.08.2015
(73) Proprietor: Zentiva, k.s., 102 37 Praha 10 (CZ)
(72) Inventor: RADL, Stanislav, 250 84 Kvetnice (CZ)
(74) Representative: Jirotkova, Ivana
(86) International application number: PCT/CZ2013/000128
(87) International publication number: WO 2014/056464

(56) References cited:
- US-A1- 2009 318 702
- US-B2- 7 176 220
- MIHIGO S O ET AL: "Total synthesis, antiprotozoal and cytotoxicity activities of rhuschalcone VI and analogs", BIOORGANIC & MEDICINAL CHEMISTRY, PERGAMON, GB, vol. 18, no. 7, 1 April 2010 (2010-04-01), pages 2464-2473, XP027027253, ISSN: 0968-0896 [retrieved on 2010-03-01] cited in the application

## Description

### Technical Field

The present invention relates to an improved method of producing elvitegravir (**I**), 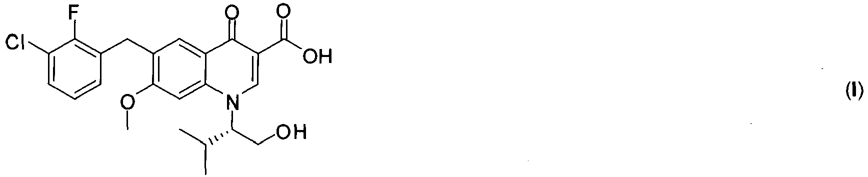 which is currently in Phase III clinical trials for the treatment of HIV infection. The drug was discovered by the company Japan Tobacco and was licensed by the company Gilead Sciences, which is carrying out the clinical development.

### Background Art

In the basic patent of the company Japan Tobacco, Inc. (US 7,176,220; EP 1,564,210; WO 2004/046115) two analogous synthetic procedures are described, starting from 2,4-difluorobenzoic acid **1**. According to the first procedure described by Scheme 1 this starting compound is first converted to benzoyl acrylate **5** in several stages. The latter is substituted with (S)-(+)-valinol **6** to the enamine **7,** which is subsequently cyclized to the quinolone **8,** whose free hydroxyl group is protected by a reaction with methyl chloroformate to the carbonate **9a.** In the next stage the carbonate reacts with 3-chloro-2-fluorobenzylzinc bromide **11** (prepared from 3-chloro-2-fluorobenzyl bromide **10** under the conditions of the Negishi coupling) in the presence of an organopalladium catalyst to the protected benzyl quinolone derivative **12a.** In the next stage the benzyl quinolone undergoes alkaline deprotection to the intermediate **13,** which is, in the last stage, subjected to a reaction with sodium methoxide to the end product Elvitegravir (Scheme 1).

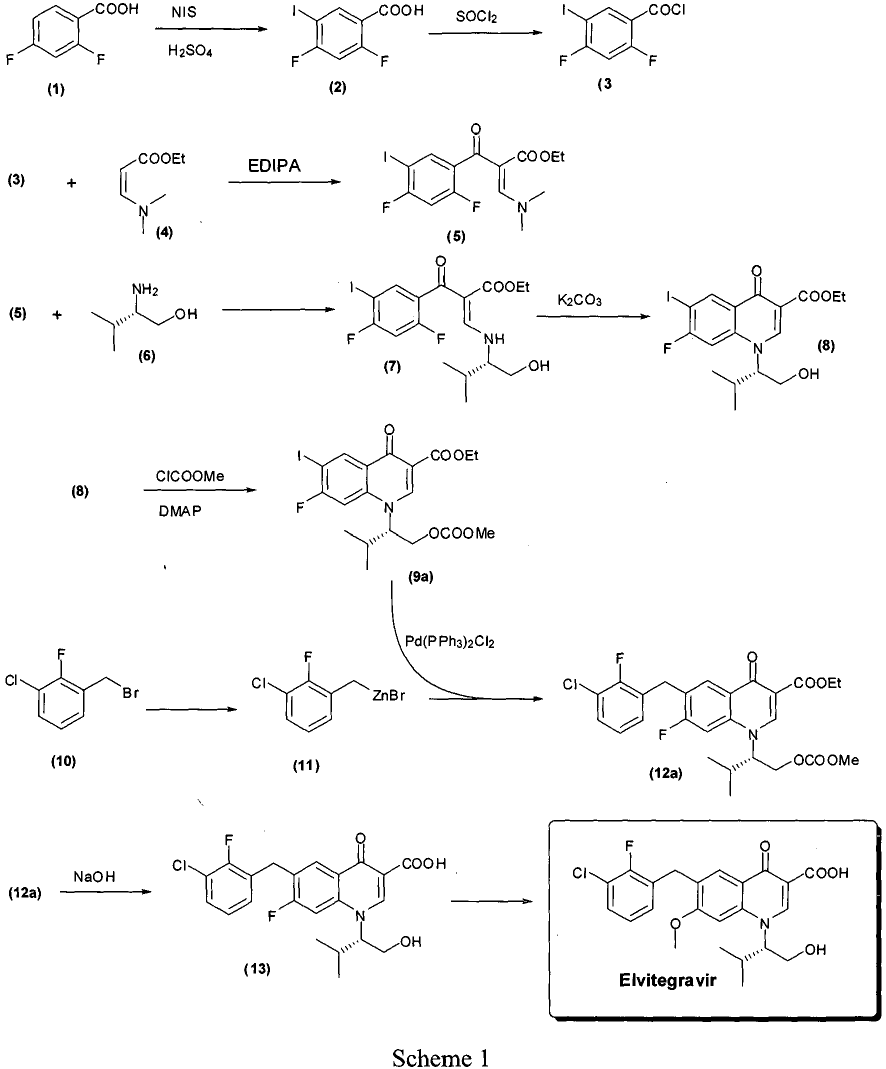

The other procedure described in the basic patent is the same as that described in Scheme 1 up to the intermediate **8,** whose hydroxyl group is protected with *tert*-butyldimethylsilyl chloride. The thus obtained protected silyl ether **9b** is further subjected to the Negishi coupling with 3-chloro-2-fluorobenzylzinc bromide **11** in the presence of bis(dibenzylidene acetone)palladium(0) and tris(2-furyl)phosphine to the protected intermediate **12b.** The remaining procedure is the same as in the previous synthetic route and includes alkaline deprotection to the hydroxy acid **13,** followed by a reaction with sodium methoxide (Scheme 2).

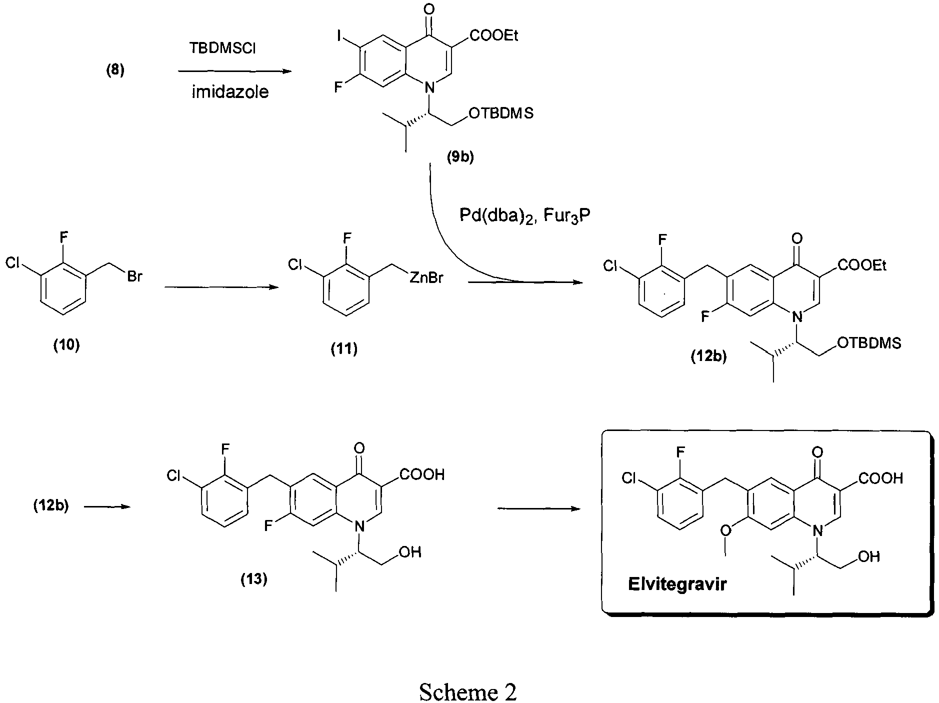

The patent application of the company Matrix Laboratories WO 2011/004389 describes synthesis that is analogous to the procedure of the basic patent, using protection of the hydroxyl by means of a tetrahydro pyranyl group (Scheme 3).

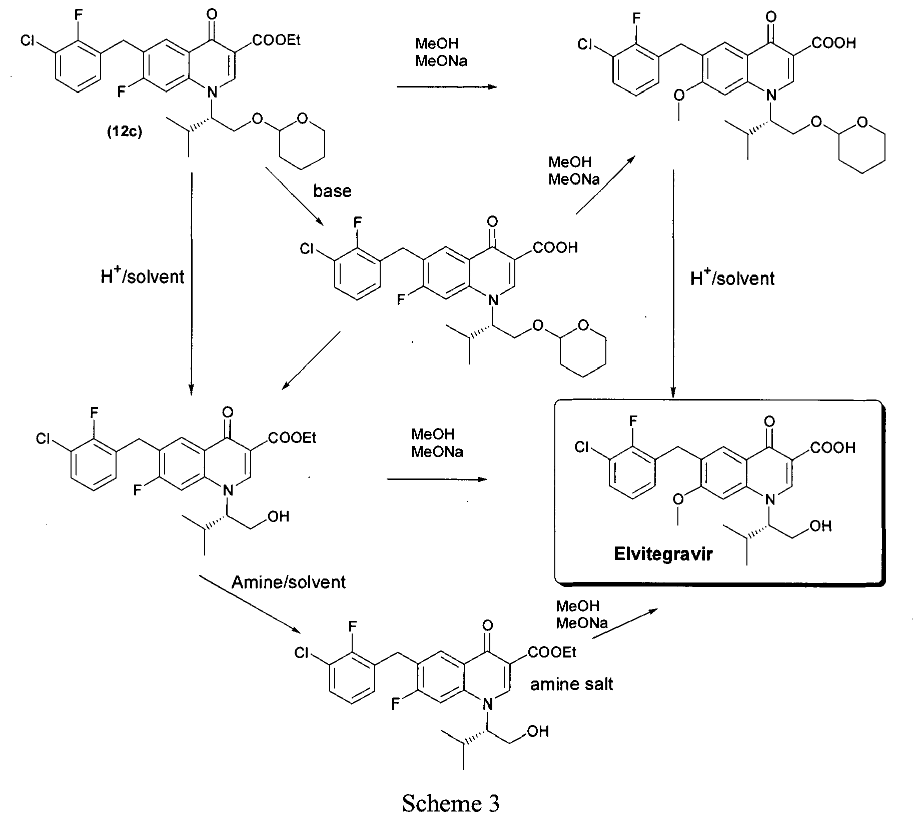

The more recent patent of the company Gilead Sciences US 7,825,252 (Scheme 3) starts from 2,4-dimethoxybenzoic acid **14.** This is converted, through a sequence of reactions, to the methyl ester **16,** whose reaction with 3-chloro-2-fluorobenzylzinc bromide **11** provides the ester **17,** which is converted to the β-ketoester **20** in the subsequent steps. Its reaction with DMF-dimethylacetal provides the benzoacrylate **21,** which further reacts with (S)-(+)-valinol **6** to provide the enamine **22.** In the next step its hydroxyl group is protected through a reaction with *tert*-butyldimethylsilyl chloride and the resulting compound **23** is subsequently cyclized to the protected quinolone derivative **24.** The last stage consists of hydrolysis of the ethyl ester as well as removal of the protecting TBDMS group (Scheme 4).

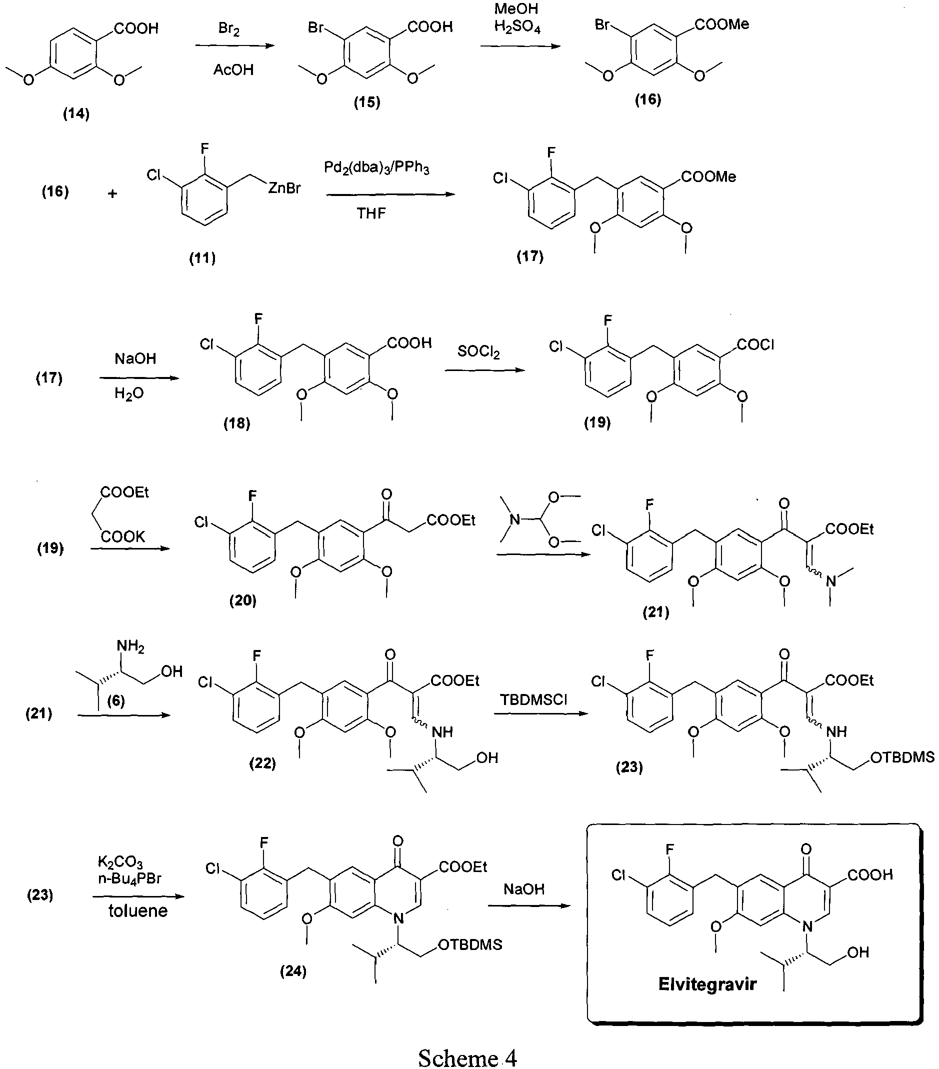

The procedure described in the process patent US 7,825,252 to Gilead Sciences uses a similar synthesis of the quinolone ring as the above mentioned procedures; however, with a different method of building up the key intermediates (Scheme 5). In the first step of this synthesis the bromoacid **15** is first converted to the magnesium salt and after subsequent addition of butyllithium the resulting salt reacts with the substituted benzaldehyde **25** to the hydroxy acid **26.** In the next step the hydroxy group is reduced with the use of triethylsilane to the acid **18.** The acid is then converted, through a reaction with carbonyldiimidazole, to the imidazole functional derivative **27,** which subsequently reacts with potassium ethylmalonate to the β-ketoester **20.** The remaining steps are the same as in the above mentioned procedures (Scheme 5).

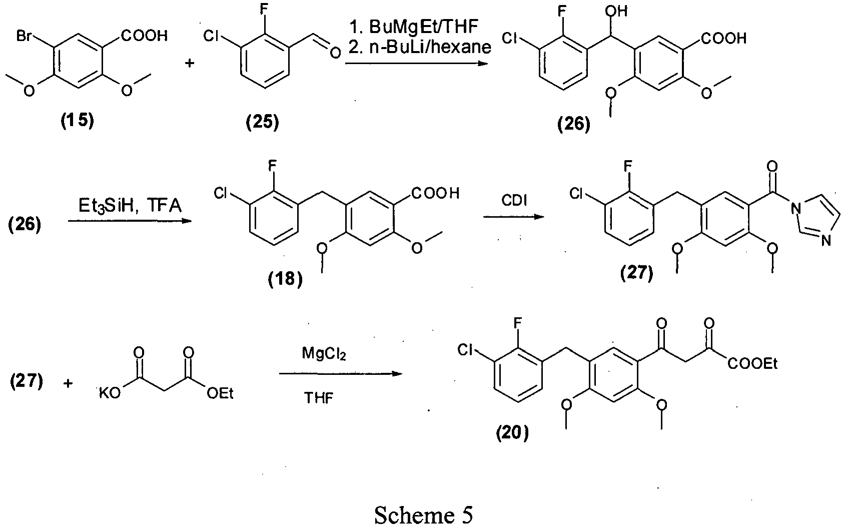

US 2009/318702 discloses a method of producing elvitegravir through a new intermediate, starting from 2,4-dimethoxybenzoic acid.

### Disclosure of Invention

This invention provides an improved method of producing intermediates of elvitegravir of the general formula **II,** 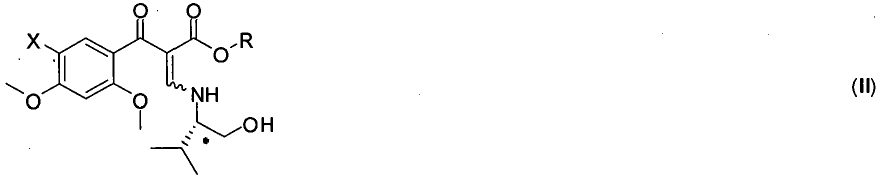 wherein X is Cl, Br, I, and wherein R is an (un)branched C1-C4 alkyl, starting from the potentially very cheap 2,4-dimethoxy acetophenone of formula **III,** produced in two stages from resorcinol, which is commonly commercially available and is produced in ton quantities.

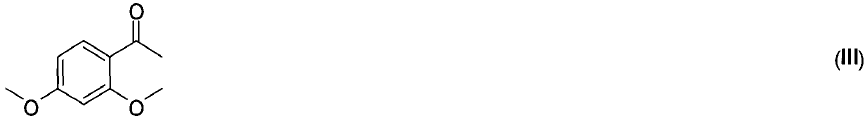

The method in accordance with this invention comprises the following steps: 2,4-dimethoxy acetophenone of formula **III** 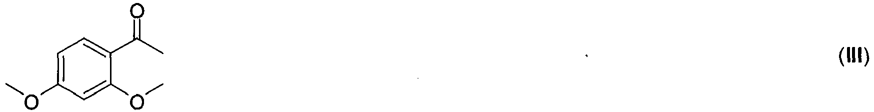 is halogenated in the nucleus, producing the compound **IV.**

The halogenation is carried out, e.g., in acetonitrile or its mixture with other solvents, at a temperature in the range of -10 to -40°C.

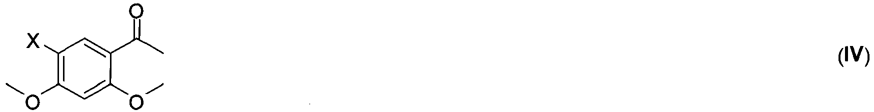

The compound IV, wherein X is Cl, Br, or I, provides, through a reaction with dialkyl carbonates in the presence of strong bases, the benzoyl acetate of general formula **V,**

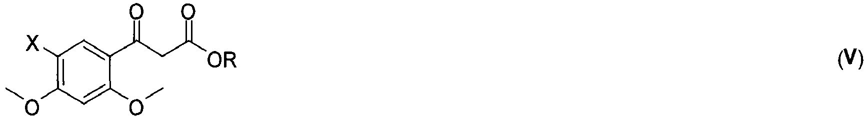

A strong base means, e.g., an alkali metal hydride, alkoxide or disilazide, preferably NaH, MeONa, *tert*-BuOK, *tert*-AmOK, potassium 3,7-dimethyl-3-octylate or sodium or potassium hexamethyldisilazide. The reaction of with dialkyl carbonates is carried out in a solvent, which is, e.g., tetrahydrofuran or toluene, or their mixtures at an increased temperature, in a preferable embodiment, at a temperature in the range from 50°C to the boiling point of the solvent or mixture used.

The compound of formula **V,** wherein X and R have the above mentioned meaning, provides, through a reaction with reagents of the general formula Me₂NC(OR¹)₂ or (Me₂N)₂COR¹, wherein R¹ is an (un)branched C1-C4 alkyl, the intermediate of the general formula **VI,**

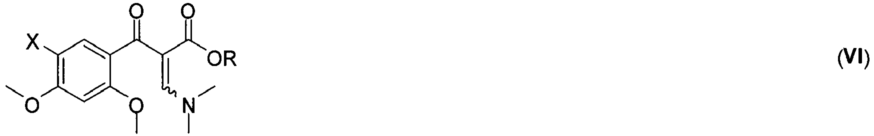

The compound of formula **VI,** wherein X and R have the above mentioned meaning, then provides the intermediate **II** through a reaction with (S)-(+)-valinol.

When a halogen atom, e.g., bromine or iodine, is introduced into the molecule of acetophenone derivatives, there is always competition between the possibility of halogenation of the acetyl group and halogenation to the nucleus. While during halogenation of acetophenones with a non-activated nucleus halogenation of the acetyl group predominantly occurs, in the case of highly activated substances, such as 2,4-dimethoxy-acetophenone, halogenation of the nucleus prevails. In spite of this, synthesis of the corresponding halo-substituted acetophenones **IV** is not easy since halogenation simultaneously occurs in the side chain, resulting in production of dihalogenated derivatives. Literature reports synthesis of the compound **IVa** by mere trituration of acetophenone **III** with *N*-bromosuccinimide without a solvent (Tetrahedron Lett. 2006, 47, 4707-4710), by bromination with tetrabutylammonium bromide in the presence of dimethyldioxirane (Lett. Org. Chem. 2009, 6, 585-587), or by bromination with bromine in acetic acid at -20 °C *(*Bioorg. Med. Chem. Lett. 2010, 18, 2464-2473). The first two of the above mentioned methods are unsuitable from the process point of view. Given that the freezing point of acetic acid is ca. 16°C, it is not surprising that solutions of acetophenone **III** in acetic acid are completely solid at a temperature around 0°C and cannot even be stirred with a mechanical stirrer. This published method is thus quite unusable. Synthesis of the iodine derivative **IVb** by direct iodination of the acetophenone **III** represents a similar situation. Again, iodination with tetrabutyl ammonium iodide in the presence of dimethyloxirane is described (Lett. Org. Chem. 2009, 6, 585-587). Another option includes iodination by iodine mediated by Selectfluor (Chem. Commun. (Cambridge, United Kingdom) 2002, 488-489), which is not commercially interesting with regard to the price of Selectfluor.

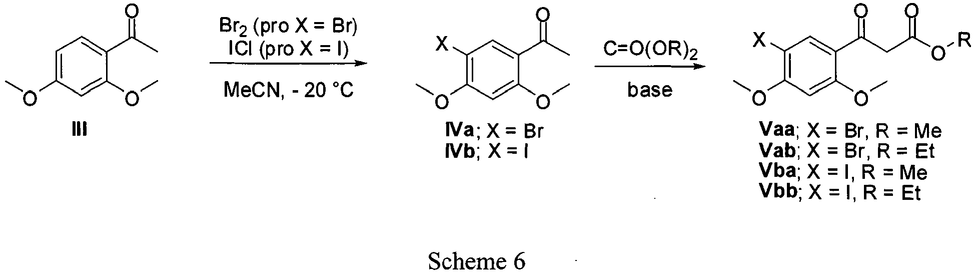

In our attempts at bromination of the compound **III** we first checked if there is not a data error in the direct bromination and whether it could not be carried out in acetic acid at 20°C. However, under these conditions a very rich mixture is formed, containing both the unreacted compound and mixtures of mono-, di- and multi-substituted derivatives. Then we focused on direct bromination in various mixed solvents containing acetic acid at -20°C. The best results were achieved with the use of a mixture of acetic acid and acetonitrile. We surprisingly found out that even better results were achieved by bromination of a suspension of the compound **III** in acetonitrile at -20 °C. We also executed iodination in a similar way with the use of ICI leading to the iodine derivative **IVb.**

Literature describes synthesis of benzoyl acetates from the corresponding acetophenones through a reaction with alkyl carbonates being catalyzed by strong bases, most frequently catalyzed by NaH. By applying the conditions described in the literature we also synthesized alkyl benzoylacetates V. We further found out that it is possible to use, as bases, also other substances in addition to NaH which are sufficiently basic and exhibit sufficient solubility in the solvent used, e.g. sodium or potassium methoxide, sodium or potassium tert-butoxide, sodium or potassium tert-amylate, and preferably sodium or potassium hexamethyldisilazide or potassium 3,7-dimethyl-3-octylate (KDMO).

In the next part of the synthesis the strategy is based on well-known synthesis procedures of the quinolone skeleton, which is also used in industry in the production of certain antibacterial quinolones. The procedure shown in Scheme 7 consists in conversion of benzoyl acetates through a reaction with orthoformates to the corresponding alkoxymethylene derivatives, or a reaction with dimethylformamide dimethylacetal (DMFDMA), or its equivalents (e.g. Bredereck's reagent) to the corresponding dimethylaminomethylene derivatives, in the present case to compounds **VI.** This reaction is usually carried out by heating of benzoyl acetates with DMFDMA alone, or in a mixture with suitable solvents (e.g. DMF or toluene) at an increased temperature. We usually used a reaction in DMFDMA alone, wherein the reaction time required for full conversion to **VI** was the shortest.

A reaction of the compounds **VI** with (S)-(+)-valinol is described for similar compounds e.g. in US 7,825,252 or WO 2009/089263, wherein the reaction is carried out without isolation of the dimethylaminomethylene intermediate from the previous reaction stage, which is carried out in toluene. The reaction can be, besides in toluene, carried in a number of other solvents, e.g. in THF, acetonitrile and a number of alcohols. The best conversion at the laboratory temperature was observed in methanol where it was already after 1 hour's stirring that the starting compound **VI** was no longer present in the reaction mixture (HPLC).

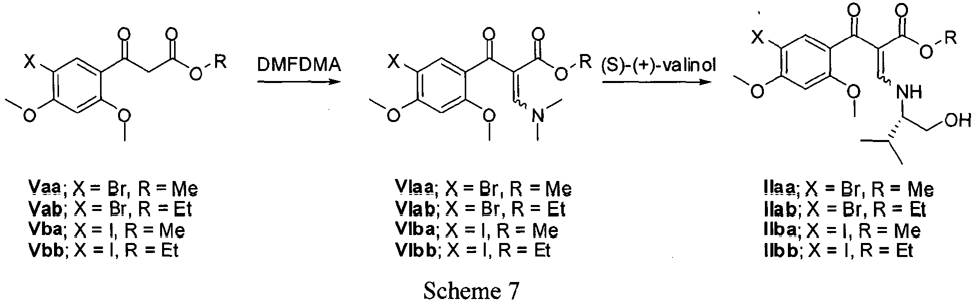

Also disclosed is a method for the preparation of elvitegravir of formula **I**, which uses the intermediate **II** according to this invention, which comprises, e.g., the following steps:
The compound **II** is converted to the protected intermediate **VII,** 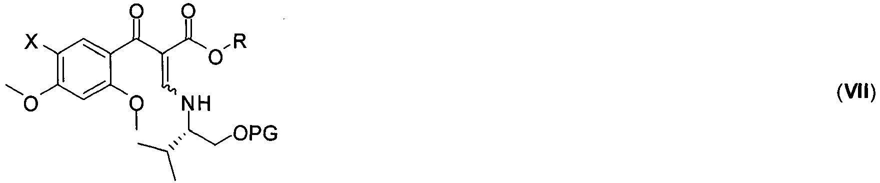 where PG may be, e.g., a tert-butyldimethylsilyl group, or other protecting group suitable for protection of the oxygen atom, as defined, e.g.,. in Protective Groups in Organic Synthesis, Third Edition. Theodora W. Greene, Peter G.M. Wuts*,*
and thus obtained intermediate is then converted, by cyclization with a suitable cyclization agent, such as with O,N-bis-trimethylsilylacetamide, to the quinoline derivative of formula **VIII,** 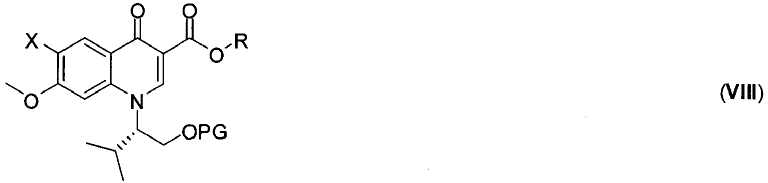 which then, through a reaction with 3-chloro-2-fluorobenzylzinc bromide, catalyzed by a suitable Pd catalyst, provides the intermediate of formula **IX,** 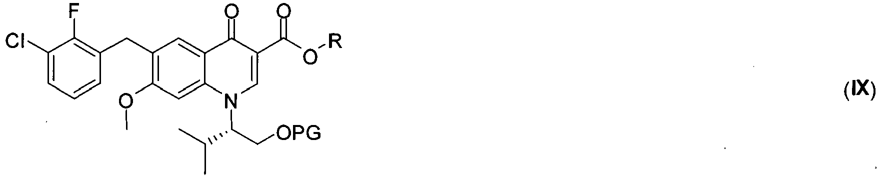 which, following deprotection and hydrolysis, is converted to elvitegravir of formula **I** by a modification of the well-known method.

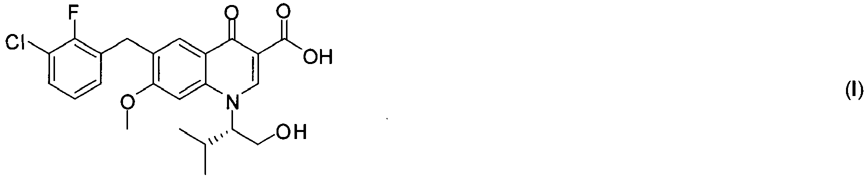

The intermediates of formula **II** can be converted to elvitegravir (**I**) using several methods described in the patent literature and summarized in the section Background Art. After protection with a suitable protecting group the obtained compounds of formula **VII** are first cyclized, e.g. by heating with BSA, to the quinolone derivative of formula **VIII.** This is then converted, through a reaction with 3-chloro-2-fluorobenzylzinc bromide catalyzed by a suitable Pd catalyst, to the protected intermediate of formula **IX**. In the subsequent steps the protecting PG group is removed and the ester is hydrolyzed to a carboxylic acid. These steps can be executed in any order. The most convenient option consists in not performing isolation and purifying of the intermediate **IX,** carrying out an acidic decomposition of the reaction mixture, during which desilylation occurs, and then performing alkaline hydrolysis of the ester and obtaining elvitegravir directly after acidification.

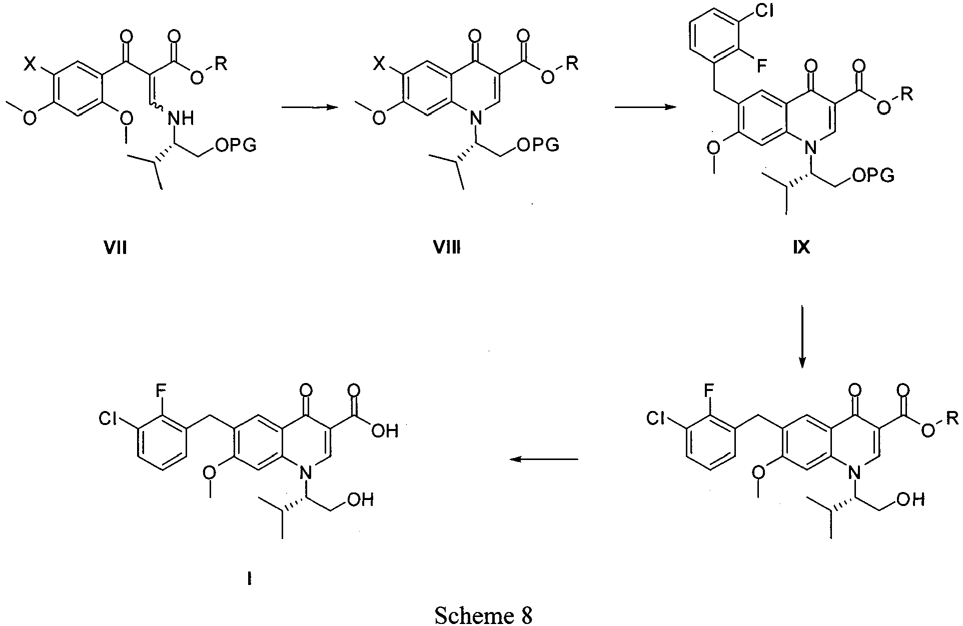

The reactions were routinely monitored with the use of HPLC in an HP 1050 device equipped with a Phenomenex Luna 5µ C18(2), 250 x 4.6 mm column and a UV detector, 227 nm. Phase A: 1.2 g of NaH₂PO₄/11 of water (pH = 3.0); Phase B: methanol.

The invention is explained in a more detailed way in the working examples below. These examples, which illustrate the improvement of the preparation method in accordance with the invention, have an exclusively illustrative character and do not limit the scope of the invention in any respect.

### Examples

### Example 1

### 1-(5-bromo-2,4-dimethoxyphenyl)ethanone (IVa)

A solution of 2,4-dimethoxyacetophenone (**III;** 180 g, 1 mol) in acetonitrile (2000 ml) was cooled to -15°C under nitrogen and bromine (200 g) was added with a linear dispenser at the same temperature during 2 hours and the mixture was stirred at this temperature for 3 h. The resulting suspension was poured onto a water-ice mixture, the insoluble fraction was aspirated, washed with water and dried in a vacuum drier at 50°C. 248.8 g (96 %) of pinkish crystals with the HPLC purity of 93.5 % was obtained, which can be used for the subsequent reaction. Crystallization of 100 g of this crude product from methanol provided 93 g (93 %) of colourless crystals with the melt. point of 140-142 °C and HPLC purity 97.7 %. ¹H NMR (250 MHz, CDCl₃) δ (ppm): 8.12 (s, 1H, H-5), 6.46 (s, 1H, H-3), 3.97 (s, 3H, MeO), 3.94(s, 3H, MeO), 2.55 (s, 3H, MeCO).

### Example 2

### 1-(5-iodo-2,4-dimethoxyphenyl)ethanone (IVb)

A solution of 2,4-dimethoxyacetophenone (**III;** 18 g, 0.1 mol) in acetonitrile (150 ml) was cooled to -20°C under nitrogen and a solution of ICl (20 g) in acetonitrile (10 ml) was added with a linear dispenser at the same temperature during 1 hour and the mixture was stirred at this temperature for 3 h. The resulting suspension was poured onto a water-ice mixture, the insoluble fraction was aspirated, washed with water and dried in a vacuum drier at 50 °C. Crystallization of the crude product from methanol provided 28.5 g (93 %) of lightly purplish crystals with the melt. point of 144-145 °C and HPLC purity of 98.9 %. ¹H NMR (250 MHz, CDCl₃) δ (ppm): 8.23 (s, 1H, H-5), 6.39 (s, 1H, H-3), 3.94 (s, 6H, MeO), 2.56 (s, 3H, MeCO).

### Example 3

### Methyl 3-(5-bromo-2,4-dimethoxyphenyl)-3-oxopropanoate (Vaa)

A charge of 60% dispersion of NaH in mineral oil (6 g) was overlaid with hexane (50 ml) under nitrogen, shortly stirred and then hexane was aspirated with a syringe through a septum. The entire procedure was repeated twice more and finally NaH was overlaid with toluene (50 ml). Dimethyl carbonate (9 g, 0.1 mol) was added to this suspension and a solution of 1-(5-bromo-2,4-dimethoxyphenyl)ethanone (**IVa;** 13 g, 50 mmol) in dry THF (120 ml) was added with a linear dispenser at the temperature of 80 °C during 1 hour and the mixture was stirred at this temperature for another 1 h. Then, the still warm reaction mixture was poured on an ice-water mixture and the resulting mixture was acidified with 10 % HCl and extracted with ethyl acetate (2 x 200 ml, 2 x 100 ml). The resulting extract was washed with brine (3 x 100 ml) and dried with MgSO₄ with an addition of Carborafin. By re-crystallization in methanol the evaporation product provided 11.2 g (71 %) of colourless crystals with the melt. point of 109-111 °C and HPLC purity of 98.8 %. ¹H NMR (250 MHz, CDCl₃) δ (ppm): 8.14 (s, 3H, H-5), 6.44 (s, 3H, H-3), 3.95 (s, 3H, MeO), 3.91 (s, 5H, MeO + CH₂), 3.67 (s, 3H, COOMe).

### Example 4

### Ethyl 3-(5-bromo-2,4-dimethoxyphenyl)-3-oxopropanoate (Vab)

Using the procedure of Example 3 with the use of diethyl carbonate the final crystallization from ethanol provided the compound **Vab** in 78 % yield with the melt. point of 81-84 °C and HPLC purity of 97.4 %. ¹H NMR (250 MHz, CDCl₃) δ (ppm): 8.04 (s, 1H, H-5), 6.48 (s, 1H, H-3), 4.22 (q, *J* = 7.1 Hz, 2H, OCH₂CH₃), 3.95 (s, 3H, MeO), 3.90 (s, 3H, MeO), 3.84 (s, 2H, CH₂), 1.31 (t, *J* = 7.1 Hz, 3H, OCH₂CH₃).

### Example 5

### Methyl 3-(5-iodo-2,4-dimethoxyphenyl)-3-oxopropanoate (Vba)

Using the procedure of Example 3 with the use of 1-(5-iodo-2,4-dimethoxyphenyl)ethanone (**IVb**) the compound **Vba** with the melt. point of 120-123°C and HPLC purity of 99.2 % was obtained in the 72 % yield. ¹H NMR (250 MHz, CDCl₃) δ (ppm): 8.34 (s, 1H, H-5), 6.37 (s, 1H, H-3), 3.95 (s, 3H, MeO), 3.91 (s, 5H, MeO + CH₂), 3.71 (s, 3H, COOMe).

### Example 6

### Ethyl 3-(5-iodo-2,4-dimethoxyphenyl)-3-oxopropanoate (Vbb)

Diethyl carbonate (10 g, 85 mmol) was added to a commercial 50% solution of KDMO in heptane (20 g, 51 mmol) and a solution of 1-(5-iodo-2,4-dimethoxyphenyl)ethanone (**IVb**; 10 g, 33 mmol) in dry THF (100 ml) was added with a linear dispenser at the temperature of 80°C during 1 hour and the mixture was stirred for another 4 h at this temperature. Then, the reaction mixture was poured on an ice-water mixture and the resulting mixture was acidified with 10% HCl and extracted with ethyl acetate (2 x 100 ml, 2 x 50 ml). The resulting extract was washed with brine (3 x 5 ml) and dried with MgSO₄ with an addition of Carborafin. By re-crystallization in methanol the evaporation product provided 8.1 g (65 %) of colourless crystals with the melt. point of 87-89 °C and HPLC purity of 97.3 %. ¹H NMR (250 MHz, CDCl₃) δ (ppm): 8.34 (s, 1H, H-5), 6.37 (s, 1H, H-3), 4.19 (q, *J* = 7.1 Hz, 2H, OCH₂CH₃), 3.95 (s, 3H, MeO), 3.91 (s, 3H, MeO), 3.89 (s, 2H, CH₂), 1.24 (t, *J* = 7.1 Hz, 3H, OCH₂CH₃).

### Example 7

### Methyl 2-(5-bromo-2,4-dimethoxybenzoyl)-3-(dimethylamino)acrylate (VIaa)

A mixture of methyl 3-(5-bromo-2,4-dimethoxyphenyl)-3-oxopropanoate (**Vaa**; 12.5 g, 39.4 mmol) and DMFDMA (30 ml) was stirred under nitrogen at 80°C for 3 hours. The reaction mixture was evaporated until dry and crystallization from methanol provided 12.7 g of yellow crystals (87 %) with the melt. point of 122-124 °C and HPLC purity of 99.1 %. ¹H NMR (250 MHz, CDCl₃) δ (ppm): 7.79 (s, 1H, -CH=), 7.64 (s, 1H, H-5), 6.41 (s, 1H, H-3), 3.89 (s, 3H, MeO), 3.80 (s, 3H, MeO), 3.51 (s, 3H, COOMe), 3.03 (s, 6H, Me₂N).

### Example 8

### Ethyl 2-(5-bromo-2,4-dimethoxybenzoyl)-3-(dimethylamino)acrylate (VIab)

Using the procedure described in Example 7 with the use of ethyl 3-(5-bromo-2,4-dimethoxyphenyl)-3-oxopropanoate (**Vab**) the compound **VIab** with the melt. point of 124-140°C and HPLC purity of 98.8 % was obtained in the yield of 92 %. ¹H NMR (250 MHz, CDCl₃) δ (ppm): 7.74 (s, 1H, -CH=), 7.64 (s, 1H, H-5), 6.41 (s, 1H, H-3), 3.91-4.01 (m, 5H, MeO + OCH₂CH₃), 3.81 (s, 3H, MeO), 3.03 (s, 6H, Me₂N), 0.97 (t, *J* = 7.1 Hz, 3H, OCH₂CH₃).

### Example 9

### Methyl 2-(5-iodo-2,4-dimethoxybenzoyl)-3-(dimethylamino)acrylate (VIba)

Using the procedure described in Example 7, starting from methyl 3-(5-iodo-2,4-dimethoxyphenyl)-3-oxopropanoate (**Vba**), methyl 2-(5-iodo-2,4-dimethoxybenzoyl)-3-(dimethylamino)acrylate (**VIba**) was obtained in the form of yellow crystals with the melt. point of 153-157 °C and HPLC purity of 97.7 % in the 83% yield. ¹H NMR (250 MHz, CDCl₃) δ (ppm): 7.97 (s, 1H, -CH=), 7.63 (s, 1H, H-5), 6.35 (s, 1H, H-3), 3.92 (s, 3H, MeO), 3.81 (s, 3H, MeO), 3.51 (s, 3H, COOMe), 3.03 (s, 6H, Me₂N).

### Example 10

### Ethyl 2-(5-iodo-2,4-dimethoxybenzoyl)-3-(dimethylamino)acrylate (VIbb)

Using the procedure described in Example 7, starting from ethyl 3-(5-iodo-2,4-dimethoxyphenyl)-3-oxopropanoate (**Vbb**), 2-(5-iodo-2,4-dimethoxybenzoyl)-3-(dimethyl-amino)acrylate **(VIbb)** was obtained in the form of yellow crystals with the melt. point of 151-156 °C and HPLC purity of 98.9 % in the 87% yield. ¹H NMR (250 MHz, CDCl₃) δ (ppm): 7.95 (s, 1H, -CH=), 7.64 (s, 1H, H-5), 6.35 (s, 1H, H-3), 3.91-4.03 (m, 5H, MeO + OCH₂CH₃), 3.81 (s, 3H, MeO), 3.03 (s, 6H, Me₂N), 0.96 (t, *J* = 7.1 Hz, 3H, OCH₂CH₃),

### Example 11

### (S)-Methyl 3-(1-hydroxy-3-methylbutan-2-ylamino)-2-(5-bromo-2,4-dimethoxybenzoyl)-acrylate (IIaa)

A solution of (S)-(+)-valinol (5 g, 48.5 mmol) in methanol (20 ml) was added to a suspension of methyl 2-(5-bromo-2,4-dimethoxybenzoyl)-3-(dimethylamino)acrylate (**VIaa;** 16.4 g, 44 mmol) in methanol (120 ml) and the mixture was stirred at the laboratory temperature for 1 h. The mixture was evaporated to 1/3 volume and after cooling the separated crystals were aspirated, washed with hexane (25 ml) and water (2 x 25 ml). After drying, 18.2 g of the compound (96 %) was obtained with the melt. point of 141-145 °C and HPLC purity of 98.5 %. ¹H NMR (250 MHz, CDCl₃) δ (ppm): 10.86 (bt, 1H, OH), 7.95 (d, 1H, -CH=), 7.52 (s, 1H, H-5), 6.41 (s, 1H, H-3), 3.92 (s, 3H, MeO), 3.63-3.78 (m, 5H, MeO + CH₂OH), 3.53 (s, 3H, COOMe), 3.05-3.12 (m, 1H, CHN), 1.92-2.00 (m, 1H, CHMe₂), 0.95-1.01 (m, 6H, CHMe₂

### Example 12

### (S)-Ethyl 3-(1-hydroxy-3-methylbutan-2-ylamino)-2-(5-bromo-2,4-dimethoxybenzoyl)acrylate (IIab)

Using the procedure described in Example 11, starting from ethyl 2-(5-bromo-2,4-dimethoxybenzoyl)-3-(dimethylamino)acrylate (**VIab**), (S)-ethyl 3-(1-hydroxy-3-methylbutan-2-ylamino)-2-(5-bromo-2,4-dimethoxybenzoyl)acrylate (**IIab**) was obtained in the 89% yield in the form of yellowish crystals with the melt. point of 113-116 °C and HPLC purity of 95.7 %. ¹H NMR (250 MHz, CDCl₃) δ (ppm): 10.87 (bt, 1H, OH), 8.09 (bd, 1H, -CH=), 7.52 (s, 1H, H-5), 6.42 (s, 1H, H-3), 3.91-4.03 (m, 5H, MeO + OCH₂CH₃), 3.72-3.90 (m, 5H, MeO + CH₂OH), 3.05-3.11 (m, 1H, CHN), 1.87-2.08 (m, 1H, CHMe₂), 0.92-1.05 (m, 9H, CHMe₂ + OCH₂CH₃).

### Example 13

### (S)-Methyl 3-(1-hydroxy-3-methylbutan-2-ylamino)-2-(5-iodo-2,4-dimethoxybenzoyl)acrylate (IIba)

Using the procedure described in Example 11, starting from ethyl 2-(5-iodo-2,4-dimethoxybenzoyl)-3-(dimethylamino)acrylate **(VIba),** (*S*)-ethyl 3-(1-hydroxy-3-methylbutan-2-ylamino)-2-(5-bromo-2,4-dimethoxybenzoyl)acrylate (**IIba**) was obtained in the 83% yield in the form of yellowish crystals with the melt. point of 115-119 °C and HPLC purity of 97.2 %. ¹H NMR (250 MHz, CDCl₃) δ (ppm): 10.87 (bt, 1H, OH), 8.09 (bd, 1H, -CH=), 7.52 (s, 1H, H-5), 6.56 (s, 1H, H-3), 3.92 (s, 3H, MeO), 3.64-3.80 (m, 5H, MeO + CH₂OH), 3.55 (s, 3H, COOMe), 3.05-3.13 (m, 1H, CHN), 1.92-2.02 (m, 1H, CHMe₂), 0.97-1.03 (m, 6H, CHMe₂).

### Example 14

### (S)-Ethyl 3-(1-hydroxy-3-methylbutan-2-ylamino)-2-(5-iodo-2,4-dimethoxybenzoyl)acrylate (IIbb)

A mixture of ethyl 3-(5-iodo-2,4-dimethoxyphenyl)-3-oxopropanoate (**Vbb**; 7.6 g, 20 mmol) and DMFDMA (10 ml) was stirred in a nitrogen atmosphere at 80°C for 4 hours. The reaction mixture was evaporated until dry, dissolved in methanol (50 ml) and a solution of *(R)-(+)-*valinol (2.5 g, 24.3 mmol) in methanol (5 ml) was added to this solution and the mixture was stirred at the laboratory temperature for 2 h. The mixture was evaporated until dry, dissolved in ethyl acetate and this solution was shaken with brine (2 x 25 ml) and dried with MgSO₄. After evaporation the evaporation product was purified by crystallization from ethanol. 8.5 g (87 %) of yellowish crystals was obtained with the melt. point of 111-116 °C and HPLC purity of 98.1 %. ¹H NMR (250 MHz, CDCl₃) δ (ppm): 10.88 (bt, 1H, OH), 7.98 (d, 1H, -CH=), 7.66 (s, 1H, H-5), 6.36 (s, 1H, H-3), 3.91-4.01 (m, 5H, MeO + OCH₂CH₃), 3.71-3.90 (m, 5H, MeO + CH₂OH), 3.05-3.16 (m, 1H, CHN), 1.81-2.02 (m, 1H, CHMe₂), 0.93-1.05 (m, 9H, CHMe₂ + OCH₂CH₃).

### Reference Example 15

### (S)-Methyl 6-bromo-1-(1-hydroxy-3-methylbutan-2-yl)-7-methoxy-4-oxo-1,4-dihydro-quinoline-3-carboxylate

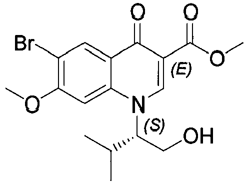

### Summary formula: C₁₇H₂₀BrNO₅

### Molecular weight: 398.25

BSA (3 g) was added to a solution of (S)-methyl 3-(1-hydroxy-3-methylbutan-2-ylamino)-2-(5-bromo-2,4-dimethoxy-benzoyl)-acrylate (**IIaa;** 1.72 g, 4 mmol) in DMF (25 ml) and the mixture was stirred at the bath temperature of 100 °C for 5 hours. Then, the mixture was diluted with water, stirred at the laboratory temperature overnight. After aspiration, washing with water and drying 1.4 g (88 %) of crystals with the melt. point of 140-1456 °C and HPLC purity of 99.4 % was obtained).

### Reference Example 16

### (S)-Methyl 6-bromo-1-(1-(tert-butyldimethylsilyloxy)-3-methylbutan-2-yl)-7-methoxy-4-oxo-1,4-dihydroquinoline-3-carboxylate

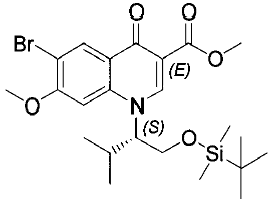

### Summary formula: C₂₃H₃₄BrNO₅Si

### Molecular weight: 512.51

A solution of (S)-methyl 6-bromo-1-(1-hydroxy-3-methylbutan-2-yl)-7-methoxy-4-oxo-1,4-dihydro-quinoline-3-carboxylate (1 g, 2.5 mmol), imidazole (1.5 g, 22 mmol) and *tert-*butyldimethylsilyl chloride (1.5 g, 10 mmol) in DMF (15 ml) was stirred at the laboratory temperature for 1 h. After addition of ethyl acetate (50 ml) the mixture was washed with water and then dried with MgSO₄. Crystallization of the evaporation product from toluene provided 0.6 g (59 %) of the compound with the melt. point of 227-235 °C.

### Example 17

### (S)-6-(3-Chloro-2-fluorobenzyl)-1-(1-hydroxy-3-methylbutan-2-yl)-7-methoxy-4-oxo-1,4-dihydroquinoline-3-carboxylic acid - elvitegravir (I)

PdCl₂(PPh₃)₂ (0.1 g) was added to a solution of (*S*)-methyl 6-bromo-1-(1-(*tert-*butyldimethylsilyloxy)-3-methylbutan-2-yl)-7-methoxy-4-oxo-1,4-dihydroquinoline-3-carboxylate (0.5 g, 1 mmol) in THF (5 ml) as a catalyst and then a 1M solution of 3-chloro-2-fluorobenzylzinc bromide in THF (1.5 ml) was added with a linear dispenser at 90°C under argon and then the mixture was stirred at this temperature for 4 h. After cooling the reaction mixture was poured onto 1M HCl (20 ml), the mixture was stirred at the laboratory temperature for 4 h and then extracted with CH₂Cl₂ (4 x 20 ml). The obtained extract was evaporated until dry without drying, dissolved in methanol (5 ml) and, after addition of a solution of NaOH (1 g) in water (10 ml), the mixture was stirred at the laboratory temperature overnight. After acidification with acetic acid and cooling, the mixture was left in a refrigerator overnight. After aspiration, 0.25 g (56 %) of the compound with the melt. point of 193-202 °C (decomp.) and HPLC purity of 96.3 % was obtained.

### Example 18

### (R)-Methyl 3-(1-hydroxy-3-methylbutan-2-ylamino)-2-(5-bromo-2,4-dimethoxybenzoyl)-acrylate) (R-IIaa)

A solution of (*R*)-(+)-valinol (1 g, 9.7 mmol) in methanol (5 ml) was added to a suspension of methyl 2-(5-bromo-2,4-dimethoxybenzoyl)-3-(dimethylamino)acrylate (VIaa; 3.7 g, 10 mmol) in methanol (30 ml) and the mixture was stirred at the laboratory temperature for 1 h. The mixture was evaporated until dry, dissolved in methyl acetate and this solution was shaken with brine (2 x 15 ml) and dried with MgSO₄. After evaporation the obtained evaporation product was re-purified by double crystallization from methanol. 3.2 g (74 %) of yellowish crystals was obtained with the melt. point of 144-146 °C with the HPLC purity of 99.6 %. ¹H NMR (250 MHz, CDCl₃) δ (ppm): 10.76 (bt, 1H, OH), 7.92 (d, 1H, -CH=), 7.50 (s, 1H, H-5), 6.45 (s, 1H, H-3), 3.94 (s, 3H, MeO), 3.82 (s, 3H, MeO), 3.61-3.71 (m, 1H, CH₂OH), 3.57 (s, 3H, COOMe), 3.03-3.14 (m, 1H, CH₂OH), 1.90-2.01 (m, 1H, CHMe₂), 0.97-1.02 (m, 6H, CHMe₂).

### Example 19

### (R)-6-(3-Chloro-2-fluorobenzyl)-1-(1-hydroxy-3 -methylbutan-2-yl)-7-methoxy-4-oxo-1,4-dihydroquinoline-3-carboxylic acid - elvitegravir (R-I)

Using the procedure described in Examples 15-17, starting from (*R*)-methyl 3-(1-hydroxy-3-methylbutan-2-ylamino)-2-(5-bromo-2,4-dimethoxybenzoyl)-acrylate) (*R*-**IIaa**), *(R)-6-(3-*chloro-2-fluorobenzyl)-1-(1-hydroxy-3-methylbutan-2-yl)-7-methoxy-4-oxo-1,4-dihydroquinoline-3-carboxylic acid - elvitegravir (*R*-**I**) was obtained in the 17% yield without isolation of intermediates in the form of colourless crystals with the melt. point of 197-201 °C.

## Claims

1. A method of producing a compound of general formula **II,** 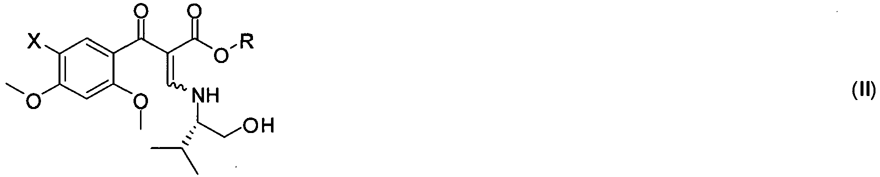 wherein X is Cl, Br, I, and wherein R is an (un)branched C₁-C₄ alkyl, **characterized in that** 2,4-dimethoxyacetophenone of formula **III** is used as the starting compound for preparation of the compound **II.**

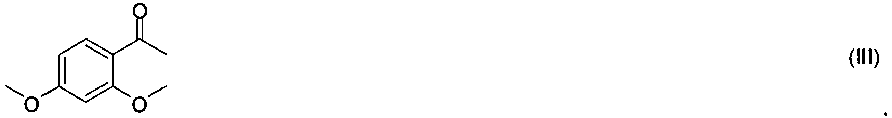

2. The method of producing according to claim 1, **characterized in that** 2,4-dimethoxyacetophenone of formula **III** 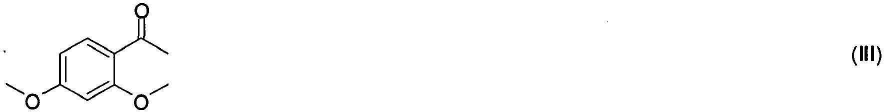 is halogenated in the nucleus, producing the compound **IV,** 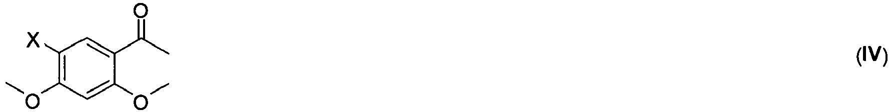 wherein X is Cl, Br, or I, which provides, through a reaction with dialkyl carbonates in the presence of strong bases, a benzoyl acetate of the general formula **V**, 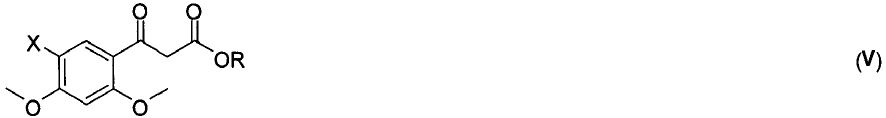 wherein X and R have the above mentioned meaning, which provides, through a reaction with reagents of the general formula Me₂NC(OR¹)₂ or (Me₂N)₂COR¹, wherein Me is a methyl group and R¹ is an (un)branched C₁-C₄ alkyl, an intermediate of the general formula **VI,** 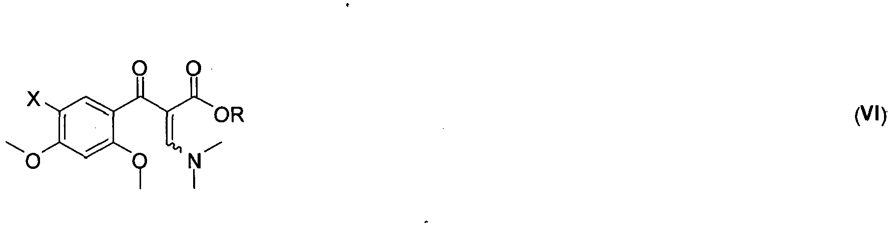 wherein X and R have the above mentioned meaning, which then provides, through a reaction with (S)-(+)-valinol, the intermediate of formula **II.**

3. The method of producing according to claim 2, **characterized in that** the halogenation is carried out in a solvent, which is acetonitrile and/or its mixtures with other solvents.

4. The method of producing according to claim 3, **characterized in that** the halogenation is carried out at the temperatures in the range of -10 °C to -40 °C.

5. The method of producing according to claim 2, **characterized in that** the strong base for the reaction with dialkyl carbonates is selected from alkali metal hydrides, alkoxides or disilazides, preferably NaH, sodium methoxide, potassium *tert-*butoxide, potassium *tert-*amylate, potassium 3,7-dimethyl-3-octylate or sodium or potassium hexamethyldisilazide.

6. The method of producing according to claim 5, **characterized in that** the reaction with dialkyl carbonates is carried out in a solvent, which is tetrahydrofuran, toluene or their mixtures at a temperature in the range from 50 °C to the boiling point of the solvent used.

7. The method of producing according to claim 2, **characterized in that** the reagent of the general formula Me₂NC(OR¹)₂ is *N,N-*dimethylformamide dimethylacetal.

8. The method of producing according to claim 2, **characterized in that** the reaction with (*S*)-(+)-valinol is carried out in a solvent which is selected from tetrahydrofuran, toluene or a C₁-C₄ (un)branched alcohol or in their mixture with other solvents.

9. The method of producing according to claim 8, **characterized in that** the reaction is carried out at temperatures in the range from 20 °C to 50 °C.

10. (*S*)-Methyl 3-(1-hydroxy-3-methylbutan-2-ylamino)-2-(5-bromo-2,4-dimethoxybenzoyl)-acrylate (**IIaa**).

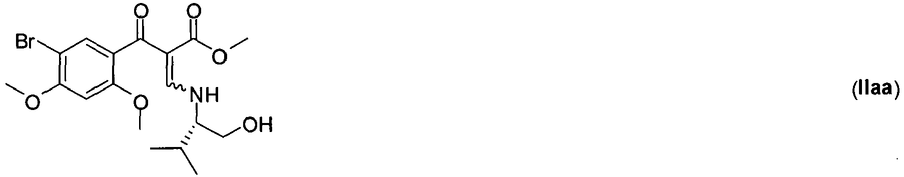

11. (*S*)-Ethyl 3-(1-hydroxy-3-methylbutan-2-ylamino)-2-(5-iodo-2,4-dimethoxybenzoyl)-acrylate (**IIba**).

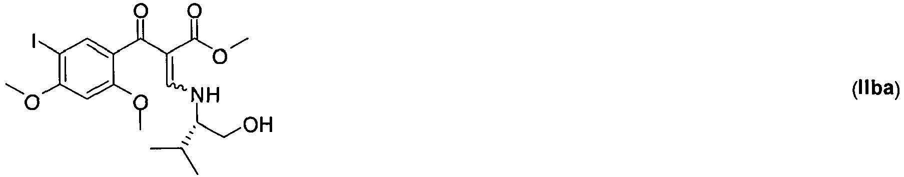

12. (*S*)-Ethyl 3-(1-hydroxy-3-methylbutan-2-ylamino)-2-(5-iodo-2,4-dimethoxybenzoyl)-acrylate (**IIbb**).

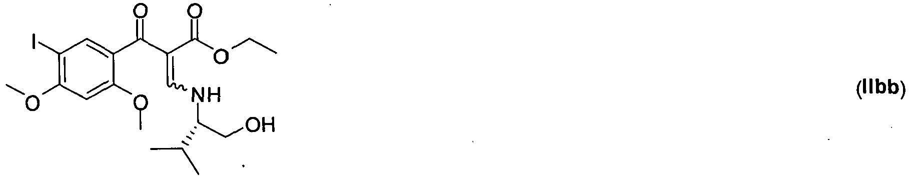

13. The method according to claims 1 and 9, **characterized in that** instead of a reaction with (*S*)-(+)-valinol a reaction with (*R*)-(+)-valinol is used and the compound **II** is prepared in the *(R)* configuration.

14. (*R*)-Methyl 3-(1-hydroxy-3-methylbutan-2-ylamino)-2-(5-bromo-2,4-dimethoxybenzoyl)-acrylate) (*R***-IIaa**).

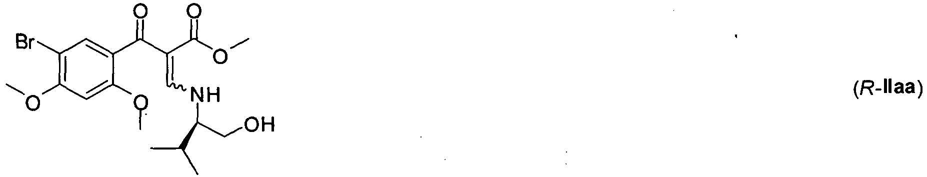

15. (*R*)-Ethyl 3-(1-hydroxy-3-methylbutan-2-ylamino)-2-(5-bromo-2,4-dimethoxybenzoyl)-acrylate) (*R***-IIab**).

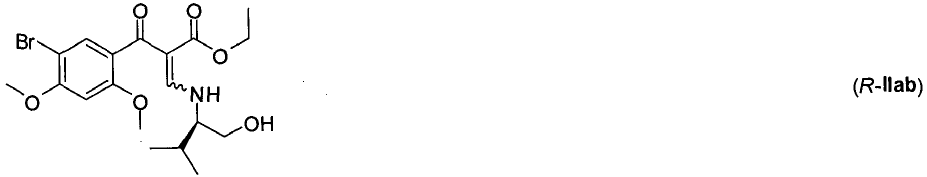

16. Use of the compounds according to claims 14 or 15 for synthesis of (*R*)-elvitegravir (*R*-**I**).

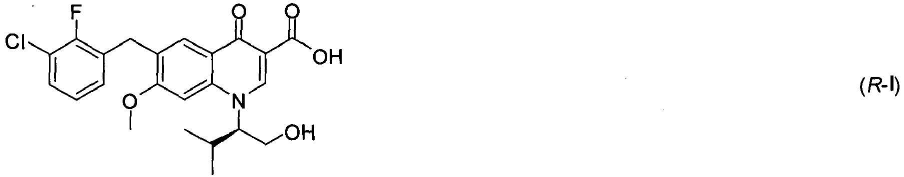

## Patentansprüche

1. Verfahren zur Herstellung einer Verbindung der allgemeinen Formel **II**, 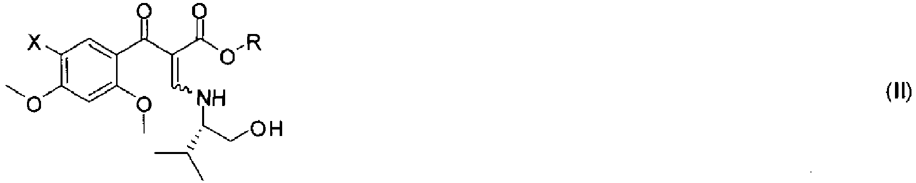 worin X Cl, Br, I ist und worin R ein (un)verzweigtes C₁-C₄-Alkyl ist, **dadurch gekennzeichnet, dass** 2,4-Dimethoxyacetophenon der Formel **III** als Ausgangsverbindung zur Herstellung der Verbindung **II** verwendet wird.

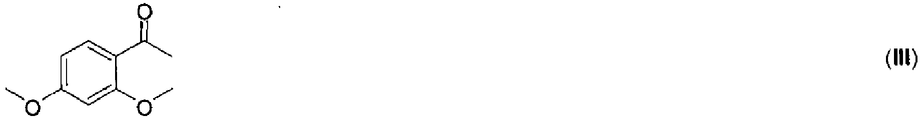

2. Verfahren zur Herstellung nach Anspruch 1, **dadurch gekennzeichnet, dass** 2,4-Dimethoxyacetophenon der Formel **III** 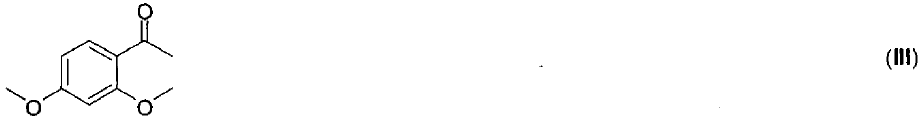 im Kern halogeniert wird, wodurch die Verbindung **IV** hergestellt wird, 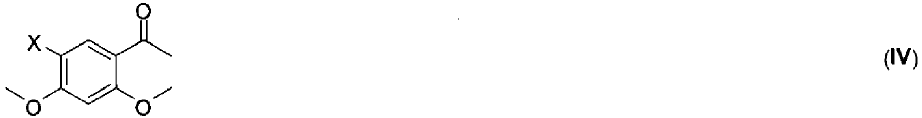 worin X Cl, Br oder I ist, welche durch Umsetzung mit Dialkylcarbonaten in Gegenwart starker Basen ein Benzoylacetat der allgemeinen Formel **V** liefert, 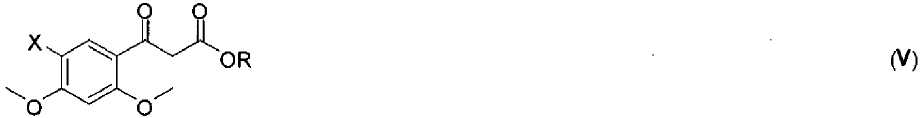 worin X und R die oben angegebene Bedeutung haben, welches durch Umsetzung mit Reagenzien der allgemeinen Formel Me₂NC(OR¹)₂ oder (Me₂N)₂COR¹, worin Me eine Methylgruppe ist und R¹ ein (un)verzweigtes C₁-C₄-Alkyl ist, ein Zwischenprodukt der allgemeinen Formel **VI** liefert, 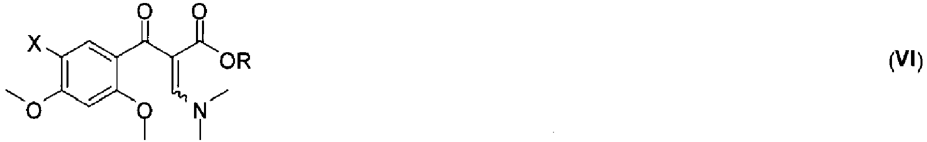 worin X und R die oben genannte Bedeutung haben, welches dann durch Umsetzung mit (S)-(+)-Valinol, das Zwischenprodukt der Formel **II** liefert.

3. Verfahren zur Herstellung nach Anspruch 2, **dadurch gekennzeichnet, dass** die Halogenierung in einem Lösungsmittel durchgeführt wird, bei dem es sich um Acetonitril und/oder dessen Mischungen mit anderen Lösungsmitteln handelt.

4. Verfahren zur Herstellung nach Anspruch 3, **dadurch gekennzeichnet, dass** die Halogenierung bei Temperaturen im Bereich von -10 °C bis -40 °C durchgeführt wird.

5. Verfahren zur Herstellung nach Anspruch 2, **dadurch gekennzeichnet, dass** die starke Base für die Umsetzung mit Dialkylcarbonaten aus Alkalimetallhydriden, Alkoholaten oder Disilaziden ausgewählt ist, vorzugsweise NaH, Natriummethoxid, Kalium-tert-butoxid, Kalium-tert-amylat, Kalium-3,7-dimethyl-3-octylat oder Natrium- oder Kaliumhexamethyl-disilazid.

6. Verfahren zur Herstellung nach Anspruch 5, **dadurch gekennzeichnet, dass** die Umsetzung mit Dialkylcarbonaten in einem Lösungsmittel, bei welchem es sich um Tetrahydrofuran, Toluol oder deren Mischungen handelt, bei einer Temperatur im Bereich von 50 °C bis zum Siedepunkt des verwendeten Lösungsmittels erfolgt.

7. Verfahren zur Herstellung nach Anspruch 2, **dadurch gekennzeichnet, dass** das Reagens der allgemeinen Formel Me₂NC(OR¹)₂ *N,N*-Dimethylformamiddimethylacetal ist.

8. Verfahren zur Herstellung nach Anspruch 2, **dadurch gekennzeichnet, dass** die Umsetzung mit (S)-(+)-Valinol in einem Lösungsmittel erfolgt, das aus Tetrahydrofuran, Toluol oder einem (un)verzweigten C₁-C₄-Alkohol ausgewählt ist, oder in einer Mischung dieser Verbindungen mit anderen Lösungsmitteln.

9. Verfahren zur Herstellung nach Anspruch 8, **dadurch gekennzeichnet, dass** die Umsetzung bei Temperaturen im Bereich von 20 °C bis 50 °C erfolgt.

10. (*S*)-Methyl-3-(1-hydroxy-3-methylbutan-2-ylamino)-2-(5-brom-2,4-dimethoxy-benzoyl)-acrylat **(IIaa).**

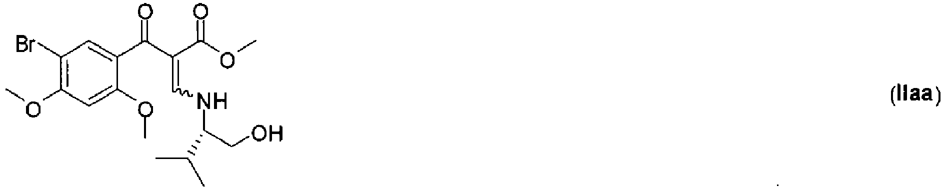

11. (*S*)-Methyl-3-(1-hydroxy-3-methylbutan-2-ylamino)-2-(5-iod-2,4-dimethoxybenzoyl)-acrylat (**IIba**).

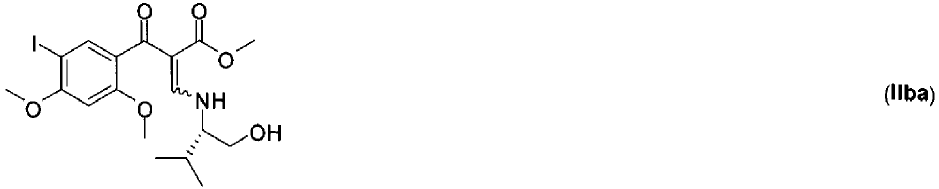

12. (*S*)-Ethyl-3-(1-hydroxy-3-methylbutan-2-ylamino)-2-(5-iod-2,4-dimethoxybenzoyl)-acrylat (**IIbb**).

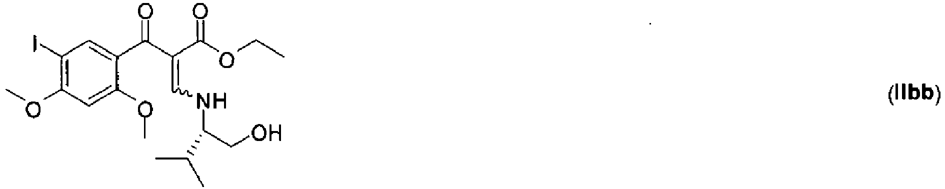

13. Verfahren nach den Ansprüchen 1 und 9, **dadurch gekennzeichnet, dass** anstelle einer Umsetzung mit (*S*)-(+)-Valinol eine Umsetzung mit (*R*)-(+)-Valinol erfolgt und die Verbindung **II** in der (*R*)-Konfiguration hergestellt wird.

14. (*R*)-Methyl-3-(1-hydroxy-3-methylbutan-2-ylamino)-2-(5-brom-2,4-dimethoxy-benzoyl)-acrylat) (**R-IIaa**).

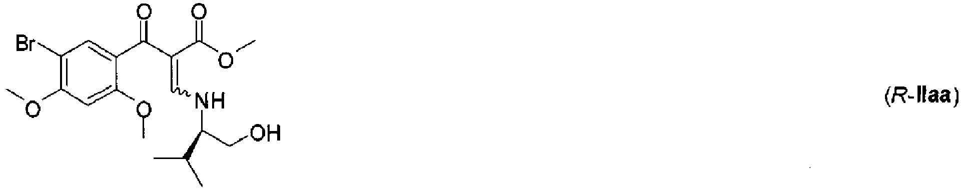

15. (*R*)-Ethyl-3-(1-hydroxy-3-methylbutan-2-ylamino)-2-(5-brom-2,4-dimethoxybenzoyl)-acrylat) (**R-IIab**).

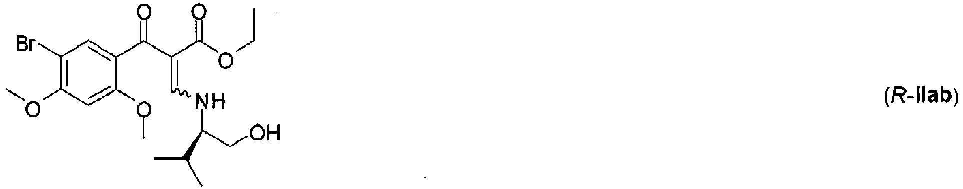

16. Verwendung der Verbindungen nach Anspruch 14 oder 15 für die Synthese von (*R*)-Elvitegravir (*R*-**I**).

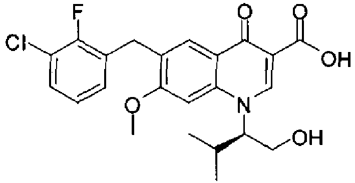

## Revendications

1. Procédé de production d'un composé de formule générale **II,** 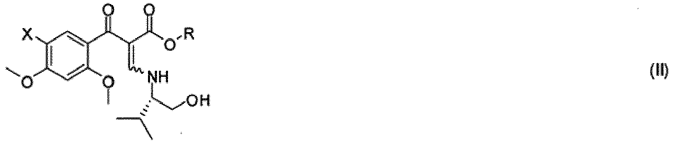 dans laquelle:
X est Cl, Br, I, et
R est un (C₁-C₄)-alkyle (non)ramifié,
**caractérisé en ce qu'**est utilisé comme composé de départ, pour la préparation du composé **II,** la 2,4-diméthoxyacétophénone de formule **III.**

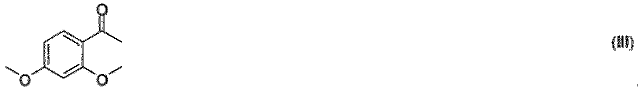

2. Le procédé de production selon la revendication 1, **caractérisé en ce que** le noyau de la 2,4-diméthoxyacétophénone de formule **III** 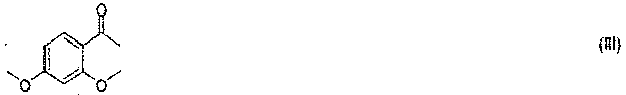 est halogéné, en produisant le composé de formule IV, 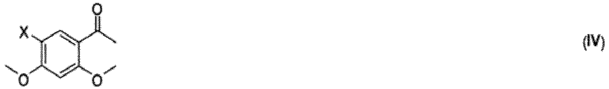 dans laquelle X est Cl, Br ou I,
ce qui donne, par réaction avec des carbonates de dialkyle en présence de bases fortes, un acétate de benzoyle de formule générale V, 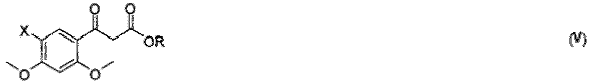 dans laquelle X et R ont la signification indiquée ci-dessus, qui fournit, par le biais d'une réaction avec des réactifs de formule générale Me₂NC(OR¹)₂ ou (Me₂N)₂COR¹, dans laquelle Me est un groupe méthyle et R¹ est un (C₁-C₄)-alkyle (non)ramifié, un intermédiaire présentant la formule générale VI, 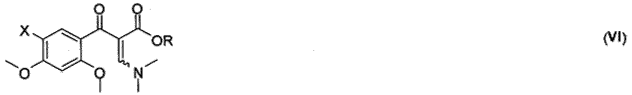 dans laquelle X et R ont la signification indiquée ci-dessus,
ce qui conduit alors, par l'intermédiaire d'une réaction avec le (S)-(+)-valinol, le composé intermédiaire de formule II.

3. Le procédé de production selon la revendication 2, **caractérisé en ce que** l'halogénation est effectuée dans un solvant qui est l'acétonitrile et/ou ses mélanges avec d'autres solvants.

4. Le procédé de production selon la revendication 3, **caractérisé en ce que** l'halogénation est effectuée à des températures dans la gamme de -10°C à -40°C.

5. Le procédé de production selon la revendication 2, **caractérisé en ce que** pour la réaction avec des carbonates de dialkyle la base forte est choisie parmi les hydrures de métaux alcalins, les alkoxydes ou disilazides, de préférence NaH, le méthoxyde de sodium, le *tert*-butoxyde de potassium, le *tert*-amylate de potassium, 3,7-diméthyl-3-octylate de potassium ou l'hexaméthyldisilazide de sodium ou de potassium.

6. Le procédé de production selon la revendication 5, **caractérisé en ce que** la réaction avec des carbonates de dialkyle est réalisée dans un solvant qui est le tétrahydrofuranne, le toluène ou leurs mélanges, à une température dans la gamme allant de 50°C au point d'ébullition du solvant utilisé.

7. Le procédé de production selon la revendication 2, **caractérisé en ce que** le réactif de formule générale Me₂NC(OR¹)₂ est le diméthylacétal de N,N-diméthylformamide.

8. Le procédé de production selon la revendication 2, **caractérisé en ce que** la réaction avec le (S)-(+)-valinol est effectuée dans un solvant qui est choisi parmi le tétrahydrofuranne, le toluène ou un alcool en (C₁-C₄) (non)ramifié ou dans leur mélange avec d'autres solvants.

9. Le procédé de production selon la revendication 8, **caractérisé en ce que** la réaction est effectuée à des températures dans la gamme de 20°C à 50°C.

10. (S)-méthyle 3-(1-hydroxy-3-méthylbutane-2-ylamino)-2-(5-bromo-2,4-diméthoxy-benzoyl)-acrylate (IIaa).

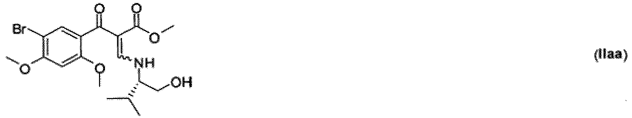

11. (S)-méthyle 3-(1-hydroxy-3-méthylbutan-2-ylamino)-2-(5-iodo-2,4-diméthoxy-benzoyl)-acrylate (IIba).

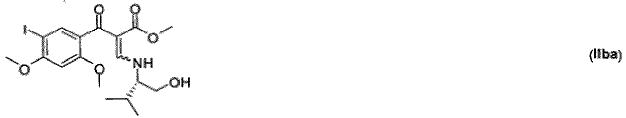

12. (S)-éthyle 3-(1-hydroxy-3-méthylbutane-2-ylamino)-2-(5-iodo-2,4-diméthoxy-benzoyl)-acrylate (IIbb).

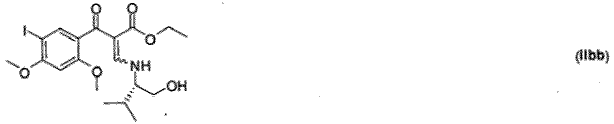

13. Le procédé selon les revendications 1 et 9, **caractérisé en ce qu'**au lieu d'une réaction avec le (S)-(+)-valinol est utilisée une réaction avec le (R)-(+)-valinol et le composé II est obtenu dans la configuration (R).

14. (R)-méthyle 3-(1-hydroxy-3-méthylbutan-2-ylamino)-2-(5-bromo-2,4-diméthoxy-benzoyl)-acrylate (R-IIaa).

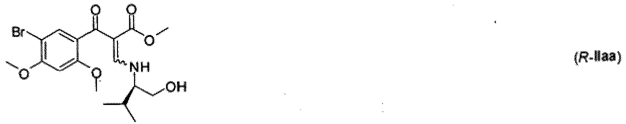

15. (R) éthyle 3-(1-hydroxy-3-méthylbutan-2-ylamino)-2-(5-bromo-2,4-diméthoxy-benzoyl)-acrylate (R-IIab).

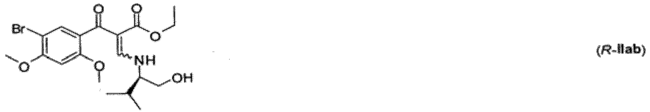

16. Utilisation des composés selon les revendications 14 ou 15 pour la synthèse de (*R*)-elvitegravir (R-I).
